# EUROPEAN PATENT SPECIFICATION

(11) **EP 1 781 853 B1**
(45) Date of publication and mention of the grant of the patent: **09.02.2011**
(21) Application number: 05780730.7
(22) Date of filing: 18.07.2005
(51) Int. Cl.: D04H 1/48

(54) **NONWOVEN LOOP SHEET AND HOOK AND LOOP FASTENING TAPE**
NICTHGEWEBTES FLAUSCHMATERIAL UND KLETTVERSCHLUSS
NON-TISSE A POILE ET FERMETURE AUTO-AGRIPPANTE

(30) Priority: 24.07.2004 KR 2004058096
(43) Date of publication of application: 09.05.2007
(73) Proprietor: Shim, Jae Hun, Chungchangnam-do 314-110 (KR)
(72) Inventor: SHIM, Jae Hun 104-708, Deoia-Apartment, Gomnaru, Chungchangnam-do, 314-110 (KR); LEE, Soo Young 1213-808, Haan-Apartment, Haan-dong, Kyunggi-do, 423-060 (KR); PARK, Tae Yaun 963-4, Hoge 1-dong, Kyunggi-do, 431-840 (KR)
(74) Representative: Hübner, Gerd
(86) International application number: PCT/KR2005/002313
(87) International publication number: WO 2006/011724

(56) References cited:
- EP-A- 0 472 992
- EP-A1- 0 472 992
- EP-A1- 0 573 277
- WO-A-03/004229
- GB-A- 2 182 685
- JP-A- 6 330 443
- US-A- 4 742 941
- US-A1- 2003 119 404
- US-A1- 2004 018 791

## Description

### Technical Field

The present invention relates to a nonwoven loop sheet which fastens on a hook tape (a hook and loop fastening tape), and use of a non-woven fabric.

### Background Art

A hook and loop fastening tape, which is a bonding unit, like an adhesive tape, a zipper, or a button, comprises a hook tape and a loop sheet, and is used as a connection unit of disposable diapers, clothes, sundries, or miscellaneous goods.

In the present invention, a nonwoven loop sheet is used as a connection unit in a disposable diaper. In the present invention, a hook tape for fastening is a tape in which hook-shaped pile is densely formed. The nonwoven loop sheet is a loop sheet in which fibre loops are densely formed so as to fasten on hooks of the hook tape. In the present invention, the loop means fibre of a nonwoven fabric, which fasten on the hook tape to generate fastening strength. If a hook side of the hook tape, on which the hooks are densely formed, comes into close contact with a fastening side of the loop sheet, the hooks fasten to the fastening side, thus two structures are connected. If a predetermined force is applied to the connected structure so as to separate the hook tape from the loop sheet, they are separated from each other with noise.

Conventionally, in miscellaneous goods including clothes, wearing apparel, and bags, and in disposable diapers for adults and infants, a fastening unit, including a hook tape and a loop sheet, is used for fastening parts of goods. The hook tape and the loop sheet of the conventional disposable diaper fasten in such a way that i) a fastening side of a woven loop tape fastens on a hook side of the hook tape, or ii) a fastening side of a nonwoven loop sheet fastens on the hook side of the hook tape. The woven loop tape is costly, thus it is unsuitable to apply to a disposable product. The nonwoven loop sheet is produced by embossing nonwoven fabrics and is low-priced. However, a lot of fluff is generated when it fastens on the hook tape and is separated from the hook tape. In the use of the hook and loop fastening tape, if fluff is formed on the nonwoven loop sheet, it is valuated as a low-grade nonwoven loop sheet. The fluff separated from the disposable diaper floats in the air in the form of dust, and may be mixed with food for infants. Thus, generation of fluff is an important item in the evaluation of quality of the nonwoven loop sheet.

With respect to fastening between the hook tape and the loop sheet of the disposable diaper, it is required that it be easy to conduct fastening and separation, repetition of fastening and separation be feasible, fastening performance be stable, they be low-priced, and little fluff be generated. However, if a surface of the unprocessed nonwoven fabric comes into contact with the hook side of the hook tape, the fastening is conducted with difficulty. The reason is as follows. 1) a fibre density of the nonwoven fabric is high and a surface thereof is smooth, thus it insufficiently fastens on the hook side. 2) a lot of fluff is generated during repetition of fastening and separation. 3) desirable fastening strength is not maintained during repetition of fastening and separation. In the present invention, the low-priced nonwoven fabric is processed at low cost so as to have loop protrusions, thereby suitable and stable fastening strength to the hook tape is assured.

The Document EP 0 472 992 A1 describes a non-woven web of fibres having at least one raised surface area which has an aperture through the web. The fibres generally adjacent to the aperture are substantially unconsolidated, i.e. not compacted together or fused. The web, having an oil capacity greater than about 500 percent by weight of oil to the weight of the web, is useful for a wiper. To form the wiper, the web is passed through a nip roller arrangement having a first roller with a plurality of unheated pins and a second roller having a plurality of corresponding orifices, to form a plurality of raised surface areas on the web, each defining an aperture where the fibres generally adjacent to the aperture are substantially unconsolidated.

The Document JP 06 330 443 A with mention in corresponding Patent Abstracts of Japan describes a method and a device for producing a sheet having three-dimensionally punched holes. The method comprises press-moulding a sheet by passing it between a pin roll having a plurality of pyramidal or conical pins on its periphery and a projection roll having a plurality of openings on its periphery so that the pins are inserted into the peripheries of the openings. The sheet has a high absorbing power and is suitable as a surface material for sanitary napkins and diapers.

The Document WO 03/004 229 A describes a non-woven fabric perforating device with a perforating roller such as a needle roller and a counter roller. A fabric to be perforated, which may be in the form of a laminate, is stretched and then guided onto the counter roller prior to be guided into a roller gap, and the perforated fabric remains on the perforating roller after leaving the gap. The fabric is thus provided with funnel- or cone-shaped perforated structures. The perforated fabric can be used for sanitary and household articles such as wiping cloths, or also medical products, surface applications, filter materials, protective clothing, geotextiles and throw-away products.

### Disclosure of Invention

### Technical Problem

In the present invention, it is observed that since a low-priced nonwoven fabric including a polypropylene nonwoven fabric has a high fibre density, it very weakly fastens on a hook tape. In order to produce a nonwoven loop sheet which readily fastens on the hook tape using the nonwoven fabric, the fibre density of a surface of the nonwoven fabric must be reduced. Additionally, it is observed that, if the nonwoven fabric fastens on the hook tape and is separated therefrom repeatedly a few times, a lot of fluff is generated. In a diaper made of the nonwoven fabric using the hook tape, it is required that desired fastening strength to the hook tape be maintained and that little fluff is generated while fastening is repeated a few times. Only when the nonwoven loop sheet can be produced at low cost, it can be commercialized. The present invention aims to provide a nonwoven loop sheet which readily fastens on a hook tape using a low-priced nowoven fabric which generates little fluff and includes a polypropylene nonwoven fabric. In the present invention, protrusions which are formed when the nonwoven fabric is holed and have predetermined fastening strength to the book tape are realized as loop protrusions. Holes are formed through the protrusions.

In accordance with the invention, the problems are solved with the subject matter of patent claims 1 and 7.

### Technical Solution

In the present invention, a nonwoven fabric is pressed and holed simultaneously, or heated, pressed, and holed simultaneously so as to form loop protrusions having a low fibre density on a surface thereof. A loop sheet of the present invention, in which a loop fastening side is formed by fastening parts of the loop protrusions having the low fiber density and the holes at tops thereof, has fair fastening strength to a hook side of the hook tape for fastening and fastening stability. Even if it is repeatedly used for fastening, its fastening strength is desirably maintained. Nonwoven fabrics have different fibre densities, degrees of generation of fluff, and fastening strengths to the hook tape depending on their types. However, they almost all have suitable fastening strength to the hook tape after formation of holes for shaping the loop protrusions.

### Advantageous Effects

The present invention provides a nonwoven loop sheet which has desirable fastening strength to a hook tape. The present invention provides a nonwoven loop sheet in which a loop fastening side is formed on a nonwoven fabric by arranging loop protrusion fastening parts on a surface of the nonwoven fabric. The present invention provides a nonwoven loop sheet which is used for fastening on a hook tape and in which a nonwoven fabric is pressed and holed simultaneously, or heated, pressed, and holed simultaneously so as to form loop protrusions having a low fibre density and holes. The present invention provides a nonwoven loop sheet which has fair fastening strength to a hook tape for fastening and fastening stability. The fastening strength is desirably maintained after fastening has been repeated a few times.

### Brief Description of the Drawings

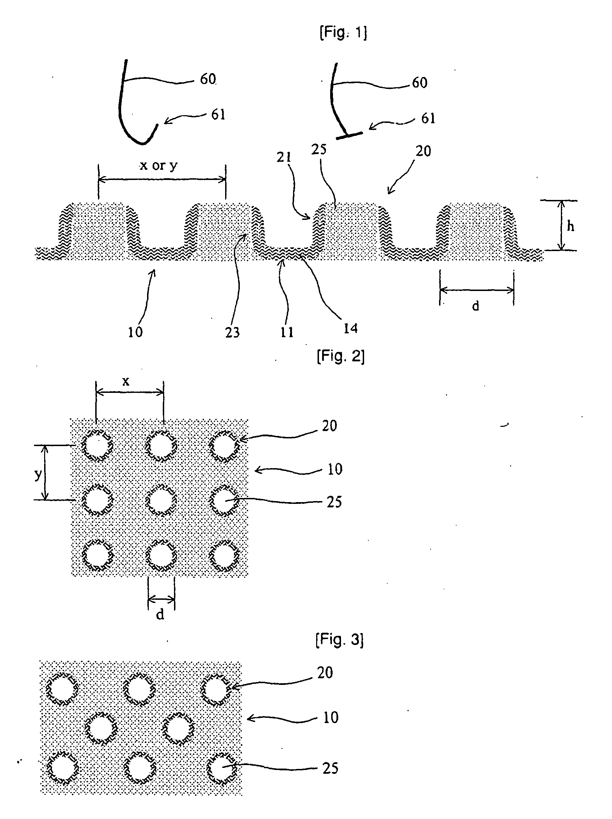

FIG. 1 is an expanded view of a loop sheet as used in the present invention, in which fibers are conceptually shown;
FIG. 2 is a plane view of the loop sheet as used in the present invention, in which the fibers are conceptually shown;
FIG. 3 is a plane view of another loop sheet as used in the present invention, in which fibers are conceptually shown;
FIG. 4 is a partially enlarged view of a device for producing a loop sheet;
FIG. 5 illustrates the formation of a nonwoven loop layer using the device;
FIG. 6 is a side view of a hard roll of the device;
FIG. 7 is a front view of a wire brush soft roll as an example of a forming roll of the device; and
FIG. 8 is a front view of an iron scrubber soft roll as another example of the forming roll of the device.

### Best Mode for Carrying Out the Invention

The present invention relates to use of a nonwoven loop sheet for fastening. The nonwoven loop sheet comprises nonwoven loop protrusions which protrude so as to be distributed over an entire nonwoven fabric to form a loop fastening side having fastening strength to a hook side of a hook tape, the nonwoven loop protrusions having holes formed at tops thereof; and hook tape connection parts which are formed around the nonwoven loop protrusions having the holes and which each have a fibre density that is lower than that of the bottom of the nonwoven fabric. In order to produce the loop sheet, a nonwoven fabric which is movably supported by a forming roll is pressed, or heated and pressed using pin punches on a surface of a pin punch roll which is heated (or maintained at ambient temperature). In a pressing process, fibres of the nonwoven fabric are stretched, the loop protrusions are formed on a surface of the nonwoven fabric, the holes are formed through tops of the loop protrusions, and the fibre density of the nonwoven fabric is lowered around the loop protrusions having the holes. The connection parts, which have the low fibre density around the loop protrusions having the holes, form a fastening side on the surface of the nonwoven fabric. The fastening side provides excellent fastening strength to the hook tape. Furthermore, the loop protrusions make the smooth surface of the nonwoven fabric uneven, thus increasing fastening strength to the hook tape. The fastening side of the nonwoven fabric used in the present invention is stably and suitably combined with the hook side of the hook tape, thereby stable fastening strength is assured. When the nonwoven loop sheet is combined with the hook tape, desirable fastening is realized and little fluff is generated during separation after fastening.

A detailed description will be given of the present invention, referring to the accompanying drawings. In a nonwoven loop sheet used in the present invention, one or more loop protrusions 20 are formed on a surface of a nonwoven fabric 10 to form a fastening side of the nonwoven fabric. The fastening side of the nonwoven fabric has fastening strength to a hook side of a hook tape, on which hooks 60 are densely formed. Holes 25 are formed through tops of the nonwoven loop protrusions. Fibre densities of parts around the loop protrusions 20 through which the holes 25 are formed are lower than those 14 of the bottom 11 of the nonwoven fabric 10, and thus connection parts 21 are formed around the loop protrusions 20 through which the holes 25 are formed. The low fibre density of the connection part positively affects connection to rakes 61 of the hooks 60. The connection parts 21 of the loop protrusions 20 form a fastening side of the nonwoven fabric. When the fastening side of the nonwoven fabric comes into close contact with the hook side of the hook tape, the rakes 61 of the hook side fasten on fibres of the connection parts 21 having low fibre density, which are positioned on a cylindrical wall of the loop protrusions 20, thereby the fastening side of the nonwoven fabric and the hook side of the hook tape desirably fasten to each other. When the loop protrusions 20 on the surface of the nonwoven fabric according to the present invention are formed, heating and pressing are conducted using pin punches 30 to form recesses 23 which correspond to the loop protrusions 20. The loop protrusions 20 of the present invention are arranged at intervals (x, y) of 0.2 35 mm, and each have a diameter (d) of 0.2 - 9 mm and a height (h) of 0.1 - 9 mm.

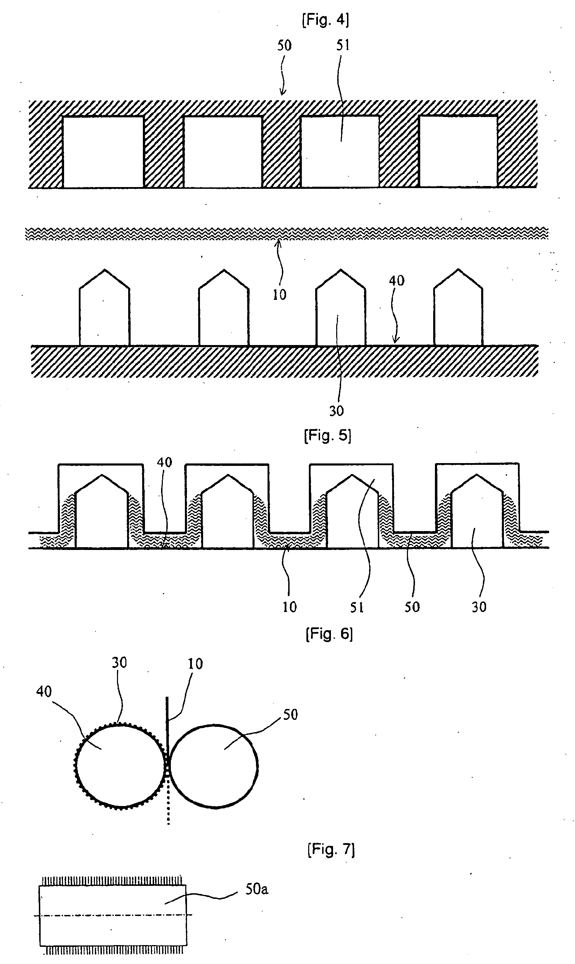

In the nonwoven loop sheet used in the present invention, the nonwoven fabric 10 is holed while it is heated and pressed to form the loop protrusions having the low fibre density on the surface of the nonwoven fabric. A punch roll 40, on a surface of which pin punches 30 are arranged, and a forming roll 50 which comes into close contact with the pin punch roll 40 for heating and pressing and movably supports the nonwoven fabric 10 to form the loop protrusions on the nonwoven fabric are provided in order to form the loop protrusions 20. Examples of the forming roll may include a hard forming roll or a soft forming roll. In FIGS. 4 to 6, shaping holes 51 corresponding to the pin punches 30 of the punch roll 40 are formed in the hard forming roll 50. The nonwoven fabric which is movably supported by the forming roll 50 is pressed by the pin punches 30 toward the center of the forming roll 50 in the shaping holes 51, thereby the loop protrusions of the nonwoven fabric are formed using the pin punches 30 and the forming roll 50, and fibres of the nonwoven fabric are stretched. The fibres of the nonwoven fabric are stretched by the pin punches 30 in the shaping holes 51 to form the loop protrusions and the holes 25 through the tops of the loop protrusions. The fibres around the protrusions through which the holes 25 are formed have low density and form the loop protrusion connection parts 21, and the fastening side of the nonwoven fabric which is formed by the loop protrusion connection parts 21 fastens well on the hook tape. Consequently, little fluff is formed on the fastening side of the nonwoven fabric.

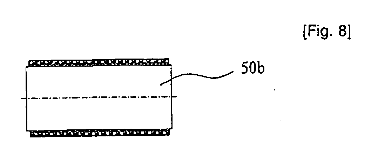

The forming roll 50 may be exemplified by the soft forming roll instead of the hard forming roll. Examples of the soft forming roll are illustrated in FIGS. 7 and 8. One of the soft forming rolls is an elastic rubber roll, elastic rubber of which is pressed toward the center thereof by pin punches while a nonwoven fabric is movably supported to stretch fibres of the nonwoven fabric to form loop protrusions and holes through tops of the loop protrusions. Another soft forming roll is a woven textile roll for nonwoven fabrics, in which a woven textile constituting a surface layer of the roll is pressed toward the center of the roll by pin punches while the nonwoven fabric is movably supported to stretch fibres of the nonwoven fabric to form loop protrusions and holes through tops of the loop protrusions (a woven textile layer is formed on the roll so that the pin punches are temporarily inserted thereinto to form marks on a surface of the roll). Still another soft forming roll is a wire brush roll 50a, in which fibres of a nonwoven fabric are pressed between wires by pin punches while a nonwoven fabric is movably supported to stretch fibres of the nonwoven fabric to form loop protrusions and holes through tops of the loop protrusions (the wires are densely formed so that the pin punches are inserted into a surface layer of the roll in the wire brush roll). Yet another soft forming roll is an iron scrubber roll 50b, in which fibres of a nonwoven fabric are pressed toward a cushion layer of an iron scrubber by pin punches while a nonwoven fabric is movably supported to stretch fibres of the nonwoven fabric to form loop protrusions and holes through tops of the loop protrusions (the iron scrubber, in which metal wires are rolled to be flattened and curled to form bubbles, is provided on a surface of the roll so that the pin punches are inserted thereinto). As described above, various types of materials may be used to produce the soft forming roll, and softness of the roll contributes to partial modification of the nonwoven fabric using the pin punches 30.

The heating temperature of the pin punch roll according to the present invention is set depending on the intrinsic heat deflection temperature of the nonwoven fabric. The heating temperature range of the pin punch roll is from ambient temperature to 200°C. In the present invention, the forming roll 50 may be heated depending on characteristics of the nonwoven fabric.

Some patterns may be printed on the loop sheet for fastening according to the present invention. A predetermined color and pattern are printed on the loop sheet for fastening, or on a laminate of the loop sheet and a reinforcing material, so as to improve qualities of products.

The loop sheet for fastening according to the present invention may be laminated with a plastic film or a nonwoven fabric as a reinforcing material at a rear side thereof. In order to laminate the loop sheet for fastening with the reinforcing material, the loop sheet for fastening and the plastic film are subjected to an extrusion lamination process, adhered to each other using an adhesive agent, laminated by heat, or laminated through an embossing process. The loop sheet for fastening and the reinforcing material may be totally or partially laminated. If the loop sheet for fastening and the reinforcing material are laminated in a process for producing a diaper, it is easy to combine the diaper with the loop sheet.

The nonwoven fabric which is used to produce the loop sheet for fastening has a weight of 10-102 g/m², and is any one of nonwoven fabrics selected from the group consisting of a polypropylene thermal-bond nonwoven fabric, a polypropylene spunbond nonwoven fabric, a polypropylene melt-blown nonwoven fabric, a bicomponent nonwoven fabric of polypropylene and polyethylene, a nonwoven fabric in which a polypropylene melt-blown nonwoven fabric and a polypropylene spunbond nonwoven fabric are combined by heat to form a structure having two or more layers, a polyethylene nonwoven fabric, a nylon (registered trademark) nonwoven fabric, a polyester nonwoven fabric, a nonwoven fabric which is produced by mixing one or more selected from the group consisting of a bicomponent grey yarn of polypropylene and polyethylene mixed with each other, a polypropylene grey yarn, a polyethylene grey yarn, a viscose grey yarn, a polyester grey yarn, and a nylon (registered trademark) grey yarn, an air-laid nonwoven fabric, an air-through nonwoven fabric, a spunlace nonwoven fabric, and a nonwoven fabric which is produced by needle-punching any one of the above-mentioned nonwoven fabrics. The above nonwoven fabrics have different fibre densities, degrees of generation of fluff, fastening strengths to the hook tape, and prices. The polypropylene-based nonwoven fabric which is cheapest and capable of being easily purchased is used in the examples of the present invention.

### Mode for the Invention

### EXAMPLE 1

Pin punches which included rolls of FIGS. 4, 5, and 6 and had a diameter of 2 mm were arranged at intervals of 2 mm in every direction. A polypropylene spun-bond nonwoven fabric of 30 g/m² was provided between a pin punch roll, which was heated to 100°C, and a forming roll, in which shaping holes were arranged so as to engage with pins, to produce a nonwoven loop sheet. In the nonwoven loop sheet, loop protrusions having fastening sides were formed on the nonwoven fabric, holes were formed through tops of the protrusions, and parts around the protrusions having the holes had a low fibre density so as to form connection parts.

### EXAMPLE 2

A flower pattern was printed on the loop sheet of example 1, and a film of 20 g/m² which included 70 parts by weight of polyethylene and 30 parts by weight of polypropylene mixed therein was extrusion laminated on a rear side of the loop sheet.

### EXAMPLE 3

An embossed nonwoven loop (L) was removed from a disposable infant diaper manufactured by a Y company, and the loop sheet for fastening (A) of example 1 was attached to the area from which the loop (L) was removed. Next, a fastening side of a hook tape (H) of the diaper was brought into close contact with a fastening side of the loop sheet for fastening (A). The hook tape (H) stably and firmly fastened to the nonwoven loop sheet (A). Fastening and separation between the loop sheet (A) and the hook tape (H) were repeated five times. Little fluff was formed on,the nonwoven fabric, and stable fastening strength was maintained during repeated fastening operations.

Fastening and separation between the conventional loop (L) and the hook tape (H) were repeated five times, and fastening and separation between the loop sheet (A) and the hook tape (H) were repeated five times. Next, the fluff which was formed on the loop sheet (A) and the loop (L) were compared using the naked eye. It was observed that the amount of fluff on the loop sheet (A) was less than that on the loop (L). The loop sheet of example 1 was produced by processing the low-priced nonwoven fabric using a simple device.

### EXAMPLE 4

An embossed nonwoven loop (L) was removed from a disposable infant diaper manufactured by Y company, and the loop sheet (B) of example 2 was attached thereto. Next, a hook tape (H) of the diaper was brought into close contact with the loop sheet (B). The hook tape (H) stably and firmly fastened to the nonwoven loop sheet (B). Fastening and separation between the loop sheet (B) and the hook tape (H) were repeated five times. Little fluff was formed on the nonwoven fabric, and stable fastening strength was maintained during repeated fastening operations.

Fastening and separation between the conventional nonwoven loop (L) and the hook tape (H) were repeated five times, and fastening and separation between the loop sheet (B) and the hook tape (H) were repeated five times. Next, the fluff which was formed on the loop sheet (B) and the loop (L) were compared using the naked eye. It was observed that the amount of fluff on the loop sheet (B) was less than that on the loop (L). The loop sheet (B) had an appearance that was better than that of the loop sheet (A) because the loop sheet (B) was printed, and was easily attached to the diaper because a film was laminated on the loop sheet (B).

### Industrial Applicability

A polypropylene-based nonwoven fabric that is cheapest among the above-mentioned nonwoven fabrics is recommended as a nonwoven fabric to be used to produce a loop sheet for the present invention. Some patterns may be printed on one side of the above-mentioned nonwoven loop sheet to be used for disposable diapers and miscellaneous goods or improve the quality thereof, and a plastic film or a nonwoven fabric as a reinforcing material may be laminated on a rear side of the nonwoven sheet so that the sheet may be easily attached to other products. Alternatively, the sheet may be used without the reinforcing material. The nonwoven loop sheet may be partially laminated with the plastic film, the nonwoven fabric, or a textile as the reinforcing material, or subjected to an embossing process to form embossments on a surface of the loop sheet. The embossments on the surface of the loop sheet increase the fastening strength to the hook tape. The loop sheet is used as a fastening part for nonwoven products, such as infant diapers, nonwoven panties, nonwoven clothes, and adult diapers.

## Claims

1. Hook and loop fastening tape, comprising:
a loop sheet of non-woven fabric (10) in which fibre loops are densely formed, and a hook tape having a hook side on which hooks (60) are densely formed, so that if the hook side of the hook tape comes into close contact with a fastening side of the loop sheet, hooks (60) of the hook tape fasten to the fastening side; and
pin-punched loop protrusions (20) protruding from the fastening side of the surface of the non-woven fabric, with holes (25) being formed through tops of the protrusions (20), and the fibre density of the non-woven fabric (10) being lowered around the protrusions (20) having the holes (25).

2. Hook and loop fastening tape according to claim 1, wherein the loop protrusions (20) having the holes are arranged at intervals (x, y) of 0,2 35 mm, and each have a diameter (d) of 0.2 - 9 mm and a height (h) of 0.1- 9 mm.

3. Hook and loop fastening tape according to claim 1, wherein the loop protrusions (20) having the holes (25) are formed by pressing using pin punches.

4. Hook and loop fastening tape according to claim 1, wherein the non-woven fabric (10) is selected from the group consisting of a polypropylene thermal-bond non-woven fabric, a polypropylene spunbond non-woven fabric, a polypropylene melt-blown non-woven fabric, a bicomponent non-woven fabric of polypropylene and polyethylene, a non-woven fabric in which a polypropylene melt-blown non-woven fabric and a polypropylene spunbound non-woven fabric are combined by heat to form a structure having two or more layers, a polyethylene non-woven fabric, a nylon (registered trademark) non-woven fabric, a polyester non-woven fabric, a non-woven fabric which is produced by mixing one or more selected from a group consisting of a bicomponent grey yarn of polypropylene and polyethylene mixed with each other, a polypropylene grey yarn, a polyethylene grey yarn, a viscose grey yarn, a polyester grey yarn, and a nylon (registered trademark) grey yarn, an air-laid non-woven fabric, an air-through non-woven fabric, a spunlace non-woven fabric, and a non-woven fabric which is produced by needle-punching any one of the above non-woven fabrics.

5. Hook and loop fastening tape according to claim 1, wherein any one of a thermoplastic plastic film and the non-woven fabric (10) is laminated on a rear side of the non-woven loop sheet.

6. Hook and loop fastening tape according to any one of claims 1 to 5, wherein a predetermined colour and a predetermined pattern are printed on the non-woven loop sheet.

7. Use of a sheet of a non-woven fabric (10), in which fibre loops are densely formed and which has pin-punched protrusions (20) protruding from a surface of the fabric (10) with holes (25) being formed through tops of the protrusions (20), with the fibre densities of the non-woven fabric (10) being lowered around the protrusions (20), as a loop sheet together with a hook tape having a hook side on which hooks (60) are densely formed in a hook and loop fastening tape.

## Patentansprüche

1. Klettverschluss, umfassend
- einen Schlaufenbogen aus einem Vliesmaterial (10), in dem Faserschlaufen dicht ausgebildet sind, sowie ein Hakenband mit einer Hakenseite, auf der Haken (60) dicht ausgebildet sind, so dass wenn die Hakenseite des Hakenbandes mit einer Verschlussseite des Schlaufenbogens in engen Kontakt kommt, sich Haken (60) des Hakenbandes an der Verschlussseite befestigen; und
- nadelgestanzte Schlaufenvorsprünge (20), die von der Verschlussseite der Oberfläche des Vliesmaterials hervorspringen, wobei in den Spitzen der Vorsprünge (20) Löcher (25) ausgebildet sind und die Faserdichte des Vliesmaterials (10) in der Umgebung der Vorsprünge (20) mit den Löchern (25) verringert ist.

2. Klettverschluss gemäß Anspruch 1 **dadurch gekennzeichnet, dass** die Schlaufenvorsprünge (20) mit den Löchern in Intervallen (x, y) von 0,2 35 mm angeordnet sind und jeweils einen Durchmesser (d) von 0,2-9 mm sowie eine Höhe (h) von 0,1-9 mm aufweisen.

3. Klettverschluss gemäß Anspruch 1, **dadurch gekennzeichnet, dass** die Schlaufenvorsprünge (20) mit den Löchern (25) mittels Splinttreibem druckgeformt sind.

4. Klettverschluss gemäß Anspruch 1, **dadurch gekennzeichnet, dass** es sich bei dem Vliesmaterial (10) um eines aus der Gruppe bestehend aus einem Polypropylen-Heißklebe-Vliesmaterial, einem Polypropylen-Spinn-Vliesmaterial, einem schmelzgeblasenen Polypropylen-Vliesmaterial, einem Zweikomponenten-Vliesmaterial aus Polypropylen und Polyethylen, einem Vliesmaterial, in dem ein schmelzgeblasenes Polypropylen-Vliesmaterial und ein Polypropylen-Spinn-Vliesmaterial durch Wärme zu einer Struktur zusammengefügt werden, die zwei oder mehr Schichten aufweist, einem Polyethylen-Vliesmaterial, einem Nylon®-Vliesmaterial, einem Polyester-Vliesmaterial, einem Vliesmaterial, das durch Vermischung von einem oder mehreren Materialien aus der Gruppe bestehend aus einem Zweikomponenten-Rohgarn aus miteinander vermischtem Polypropylen und Polyethylen, einem Polypropylen-Rohgarn, einem Polyethylen-Rohgarn, einem Viskose-Rohgarn, einem Polyester-Rohgarn und einem Nylon®-Rohgarn hergestellt wird, einem luftgelegten Vliesmaterial, einem luftgebundenen Vliesmaterial, einem wasserstrahlverfestigten Vliesmaterial oder einem Vliesmaterial, das durch Vemadelung eines der oben genannten Vliesmaterialien hergestellt wird, handelt.

5. Klettverschluss gemäß Anspruch 1, **dadurch gekennzeichnet, dass** eine beliebige thermoplastische Kunststofffolie und das Vliesmaterial (10) auf eine Rückseite des Vlies-Schlaufenbogens auflaminiert werden.

6. Klettverschluss gemäß einem der Ansprüche 1 bis 5, **dadurch gekennzeichnet, dass** eine vorbestimmte Farbe und ein vorbestimmtes Muster auf den Vlies-Schlaufenbogen aufgedruckt werden.

7. Verwendung eines Bogens aus einem Vliesmaterial (10), bei dem Faserschleifen dicht ausgebildet sind und das ausgetriebene Vorsprünge (20) aufweist, die von einer Oberfläche des Materials (10) hervorspringen, wobei in Spitzen der Vorsprünge (20) Löcher (25) ausgeformt sind und die Faserdichten des Vliesmaterials (10) in der Umgebung der Vorsprünge (20) verringert sind, als ein Schlaufenbogen zusammen mit einem Hakenband, das eine Hakenseite aufweist, auf der Haken (60) dicht ausgebildet sind, in Form eines Klettverschlusses.

## Revendications

1. Bande de fermeture velcro comprenant :
une feuille de boucles en tissu non tissé (10) dans laquelle les boucles de fibre sont formées de manière dense, et une bande de crochets ayant un côté de crochets sur lequel des crochets (60) sont formés de manière dense, de sorte que si le côté de crochets de la bande de crochets vient en contact étroit avec le côté de fixation de la feuille de boucles, les crochets (60) de la bande de crochets se fixent sur le côté de fixation ; et
des saillies de boucles découpées à l'emporte-pièce (20) faisant saillie du côté de fixation de la surface du tissu non tissé, avec des trous (25) qui sont formés à travers les parties supérieures des saillies (20), et la densité de fibre du tissu non tissé (10) étant inférieure autour des saillies (20) comprenant les trous (25).

2. Bande de fermeture velcro selon la revendication 1, dans laquelle les saillies de boucles (20) comprenant les trous sont agencées à intervalles (x, y) de 0,235 mm et chacun a un diamètre (d) de 0,2 - 9 mm et une hauteur (h) de 0,1 - 9 mm.

3. Bande de fermeture velcro selon la revendication 1, dans laquelle les saillies de boucles (20) comprenant les trous (25) sont formées par compression en utilisant des emporte-pièce.

4. Bande de fermeture velcro selon la revendication 1, dans laquelle le tissu non tissé (10) est choisi dans le groupe comprenant un tissu non tissé thermosoudé en polypropylène, un tissu non tissé filé-lié en polypropylène, un tissu non tissé obtenu par fusion-soufflage en polypropylène, un tissu non tissé bicomposé de polypropylène et de polyéthylène, un tissu non tissé dans lequel un tissu non tissé obtenu par fusion-soufflage en polypropylène et un tissu non tissé filé-lié en polypropylène sont combinés à la chaleur pour former une structure ayant deux couches ou plus, un tissu non tissé en polyéthylène, un tissu non tissé en nylon (marque déposée), un tissu non tissé en polyester, un tissu non tissé qui est produit en mélangeant un ou plusieurs éléments sélectionnés dans le groupe se composant d'un fil écru bicomposé de polypropylène et de polyéthylène mélangés entre eux, un fil écru en polypropylène, un fil écru en polyéthylène, un fil écru en viscose, un fil écru en polyester et un fil écru en nylon (marque déposée), un tissu non tissé air-laid, un tissu non tissé à passage d'air, un tissu non tissé lacé par filage, un tissu non tissé qui est produit par aiguilletage de l'un quelconque parmi les tissus non tissés ci-dessus.

5. Bande de fermeture velcro selon la revendication 1, dans laquelle l'un quelconque parmi un film plastique thermoplastique et le tissu non tissé (10) est déposé en couche sur un côté arrière de la feuille de boucles non tissée.

6. Bande de fermeture velcro selon l'une quelconque des revendications 1 à 5, dans laquelle une couleur prédéterminée et un modèle prédéterminé sont imprimés sur la feuille de boucles non tissée.

7. Utilisation d'une feuille de tissu non tissé (10), dans laquelle des boucles de fibre sont formées de manière dense et qui a des saillies aiguilletées (20) faisant saillie d'une surface du tissu (10) avec des trous (25) qui sont formés à travers les parties supérieures des saillies (20) avec les densités de fibre du tissu non tissé (10) qui sont inférieures autour des saillies (20), étant donné qu'une feuille de boucles conjointement à une feuille de crochets a un côté de crochets sur lequel des crochets (60) sont formés de manière dense dans une bande de fermeture velcro.
